# EUROPÄISCHE PATENTSCHRIFT

(11) **EP 3 079 563 B1**
(45) Veröffentlichungstag und Bekanntmachung des Hinweises auf die Patenterteilung: **04.10.2023**
(21) Anmeldenummer: 14825091.3
(22) Anmeldetag: 20.11.2014
(51) Int. Cl.: A61B 5/00, A61B 1/04

(54) **VORRICHTUNG MIT EINER RAMAN-SONDE UND EIN VERFAHREN UNTER VERWENDUNG DIESER VORRICHTUNG**
DEVICE WITH A RAMAN PROBE, AND METHOD USING SAID DEVICE
DISPOSITIF MUNI D'UNE SONDE RAMAN ET PROCÉDÉ UTILISANT LEDIT DISPOSITIF

(30) Priorität: 09.12.2013 DE 102013020703; 14.04.2014 DE 102014005617
(43) Veröffentlichungstag der Anmeldung: 19.10.2016
(73) Patentinhaber: Friedrich-Schiller-Universität Jena, 07743 Jena (DE); Leibniz-Institut für Photonische Technologien e.V., 07745 Jena (DE)
(72) Erfinder: DOCHOW, Sebastian, 07745 Jena (DE); POPP, Jürgen, 07751 Kunitz (DE)
(74) Vertreter: Donath, Dirk
(86) Internationale Anmeldenummer: PCT/DE2014/000597
(87) Internationale Veröffentlichungsnummer: WO 2015/085978

(56) Entgegenhaltungen:
- WO-A1-2013/067996
- US-A- 6 006 001
- US-A1- 2008 119 832
- US-A1- 2009 021 724
- US-A1- 2009 231 578
- INES LATKA ET AL: "Fiber optic probes for linear and nonlinear Raman applications - Current trends and future development", LASER & PHOTONICS REVIEWS, Bd. 7, Nr. 5, 15. Januar 2013 (2013-01-15) , Seiten 698-731, XP55176249, ISSN: 1863-8880, DOI: 10.1002/lpor.201200049
- CHETAN A. PATIL ET AL: "A clinical instrument for combined raman spectroscopy-optical coherence tomography of skin cancers", LASERS IN SURGERY AND MEDICINE, Bd. 43, Nr. 2, 7. Februar 2011 (2011-02-07), Seiten 143-151, XP55178021, ISSN: 0196-8092, DOI: 10.1002/lsm.21041
- LATKA INES ET AL: "Development of a fiber-based Raman probe for clinical diagnostics", CLINICAL AND BIOMEDICAL SPECTROSCOPY AND IMAGING II, SPIE, 1000 20TH ST. BELLINGHAM WA 98225-6705 USA, Bd. 8087, Nr. 1, 9. Juni 2011 (2011-06-09) , Seiten 1-8, XP060015296, DOI: 10.1117/12.889924 [gefunden am 1901-01-01]
- MANU JAIN ET AL: "Multiphoton Microscopy in the Evaluation of Human Bladder Biopsies", ARCHIVES OF PATHOLOGY & LABORATORY MEDICINE, Bd. 136, Nr. 5, 1. Mai 2012 (2012-05-01), Seiten 517-526, XP55178022, ISSN: 0003-9985, DOI: 10.5858/arpa.2011-0147-OA

## Beschreibung

Die vorliegende Erfindung betrifft eine Vorrichtung mit einer RamanSonde und ein Verfahren unter Verwendung dieser Vorrichtung zur Erzeugung und zur Detektion von Red-Flag- Strahlung, wie bspw. Fluoreszenzstrahlung, und eines Raman- Spektrums, insbesondere zur endoskopischen *in vivo-* Untersuchung von biologischem Gewebe, wobei eine Klassifizierung nach Gewebetyp erfolgt.

Laser-Scanning- Mikroskope, insbesondere Multiphotonen- Fluoreszenz-Mikroskope (meist Zwei-Photonen- Fluoreszenz) oder faserbasierte Fluoreszenzendoskope werden u.a. für die Detektion verschiedener biologischer Gewebe eingesetzt.

Bei der herkömmlichen Fluoreszenzmikroskopie wird in einem fluoreszierenden Molekül ein Elektron durch die Absorption jeweils eines Photons angeregt, also in einen höheren Energiezustand versetzt.

Die Methode der Fluoreszenzbildgebung erfordert meistens einen Farbstoff, welcher in irgendeiner geeigneten Weise im Zielgebiet eines biologischen Gewebes (bspw. dem Tumorgewebe) angereichert werden muss. In manchen Fällen kann auch die so genannte Autofluoreszenz des biologischen Gewebes genutzt werden, also die Fluoreszenz, die in verschiedenen Geweben ohne die Zugabe eines Farbstoffes auftritt.

Fluoreszenz hat den Vorteil einer hohen Quanteneffizienz des Prozesses, wodurch eine Echtzeit- Bildgebung des betrachteten biologischen Gewebes möglich ist.

Standard sind hier Bildwiederholfrequenzen bis 60 Hz. Dadurch ist die schnelle Erzeugung eines Übersichtsbildes, bspw. von biologischem Gewebe, möglich, wobei voneinander verschiedene Gewebeareale/- typen bildlich unterschiedlich dargestellt werden können.

Bei der Zwei-Photonen- Fluoreszenzmikroskopie wird die Anregung des Elektrons durch die gleichzeitige Absorption zweier Photonen hervorgerufen (Zwei-Photonen- Absorption). Auch eine Anregung mit drei oder mehr gleichzeitig eintreffenden Photonen ist möglich.

Mit Hilfe eines starken, fokussierten Laserstrahls werden beim Multiphotonen-Fluoreszenz- Mikroskop nichtlineare optische Effekte erzeugt, die auf dem Zusammenspiel mehrerer gleichzeitig in einem Molekül eintreffenden Photonen beruhen. Die Stärke des erzeugten Signals steigt daher nicht linear mit der Zahl der eingestrahlten Photonen, sondern mit dem Quadrat (bei Zwei-Photonen- Effekten) oder der dritten Potenz (bei Drei-Photonen- Effekten).

Mit der Multiphotonen- Fluoreszenzmikroskopie können tiefere Bereiche, bspw. von 200 µm bis zu 1000 µm erreicht werden, wodurch Fluoreszenzaufnahmen von lebenden, biologischen Geweben möglich sind, die anderweitig für die Bildgebung unerreichbar sind. (Die konfokale Laser-Scanning- Mikroskopie weist bspw. eine Eindringtiefe je nach Präparat von nur 50 bis 80 µm auf).

Mittels 2-Photonen- Laser- Mikroskopie können bspw. die Tumorzellen in der Kokultur anhand der GFP-Fluoreszenz identifiziert werden Martin [Edelmann, Rudolf Maria Huber und Albrecht Bergner: "Tumoranalyse in einem dreidimensionalen Kokulturmodell"; Pneumologie 02/2009, Seite 63 - 92; Offenbarung der Publikation online unter: www.thieme-connect.com/ejournals/abstract/10.1055/s-0029-1202420)].

Mit der Multiphotonen- Fluoreszenzmikroskopie kann ausgewertet werden, ob der Farbstoff bzw. das biologische Gewebe eine Fluoreszenz zeigt, was jedoch in den meisten Fällen nicht spezifisch genug ist, um zu erkennen, ob es sich um tumoröses Gewebe handelt, oder zu charakterisieren, um welchen Tumortyp es sich handelt.

Daher werden in vielen Fällen mehrere Farbstoffe verwendet oder, im Falle der Autofluoreszenz, mehrere Fluorophore angeregt, um die Spezifität zu erhöhen. Dies gelingt jedoch nur begrenzt, da bspw. verschiedene Färbemethoden für den gleichen Gewebetyp gefunden werden müssen.

Der Nachteil der bekannten Fluoreszenztechniken zur Untersuchung von biologischem Gewebe besteht somit darin, dass man durch sie nur erkennen kann, dass bspw. eine Gewebeveränderung vorliegt. Diese Erkennung kann, wie zuvor stehend bereits beschrieben, sehr schnell an Hand eines Fluoreszenzübersichtsbildes gewonnen werden. Diese Technik der Bereitstellung eines schnellen Übersichtsbildes, bspw. zur Anzeige, ob eine Veränderung im biologischen Gewebe vorliegt oder nicht, wird als "Red-Flag"- Technik bezeichnet.

Die Strahlung, die bei dieser Technik verwendet wird, ist im Folgenden als "Red-Flag- Strahlung" bezeichnet.

Nachteilig an den bekannten Fluoreszenztechniken (Fluoreszenzmikroskopie und -endoskopie) ist somit, dass man mit ihnen nicht klassifizieren / charakterisieren kann, um welche Gewebeveränderung es sich konkret handelt (bspw. ob ein Tumorgewebe vorliegt und wenn "ja", welche Art von Tumorgewebe gegeben ist).

Ansatzpunkte für eine gegenüber der Fluoreszenzmikroskopie und -endoskopie spezifischen Klassifizierung von biologischem Gewebe bietet die Raman- Spektroskopie.

Diese Raman- Spektroskopie basiert auf der inelastischen Lichtstreuung von Photonen, welche detaillierte, chemische Informationen über die Probe enthalten, anhand derer eine Klassifikation vorgenommen werden kann.

Bei der Raman- Spektroskopie wird die zu untersuchende Materie mit monochromatischem Licht bestrahlt, üblicherweise aus einem Laser. Im Spektrum des an der Probe gestreuten Lichts werden neben der eingestrahlten Frequenz (Rayleigh- Streuung) noch weitere Frequenzen beobachtet, die auf einer Wechselwirkung des Lichtes mit der Materie, dem sogenannten Raman- Effekt beruhen.

Die Frequenzunterschiede zum eingestrahlten Licht entsprechen den für das Material charakteristischen Energien von Rotations-, Schwingungs-, Phonon- oder Spin-Flip- Prozessen.

Da die Wellenlänge des Lichts, d. h. seine Farbe, von der Energie des Lichtes abhängt, bewirkt dieser Energieübertrag eine Verschiebung der Wellenlänge des gestreuten Lichtes gegenüber dem eingestrahlten Licht, die sogenannte Raman- Verschiebung.

Aus dem erhaltenen Spektrum ("Stokes- Seite" bzw. "Anti-Stokes- Seite" des Spektrums) lassen sich, ähnlich dem Spektrum der Infrarotspektroskopie, Rückschlüsse auf die untersuchte Substanz ziehen.

Neben der klassischen Raman- Spektroskopie existieren noch einige Varianten und Weiterentwicklungen. Dazu gehören:
- auf der nichtlinearen Raman- Streuung basierende Verfahren, wie bspw. die kohärente Anti-Stokes-Raman- Streuung (s.g. CARS),
- auf der oberflächenverstärkten Raman- Streuung basierende Verfahren (s.g. SERS) und
- die spitzenverstärkten Raman- Spektroskopie (s.g. TERS) als Kombination aus SERS und Rasterkraftmikroskopie (s.g. AFM).

Die Raman- Spektroskopie erlaubt auch Aussagen über wässrige Systeme, welche über Infrarot- Spektroskopie schwer zugänglich sind. So sind nicht nur abiotische, sondern auch biotische Systeme der Analyse zugänglich. Es ist prinzipiell sogar möglich, einzelne Spezies von Bakterien oder biologisches Gewebe mittels Raman- Spektroskopie zu unterscheiden.

Durch die schwache Quanteneffizienz bei der Raman- Spektroskopie sind jedoch lange Messzeiten notwendig. Die Dauer der Aufnahme eines s.g. Raman-Spektrums liegt im Bereich von mehreren 100 Millisekunden, was den Anforderungen einer schnellen Bildgebung nicht gerecht wird.

Weiterhin müssen diese Art von Sonden gefiltert werden um die intrinischen Untergründe zu unterdrücken. Dies geschieht zum Beispiel durch separat gefertigte Filter, welche dann auf die Sonde gebracht werden (siehe dazu bspw. US 6,222,970 B1) oder durch Beschichtungen des Faserbündels (siehe dazu bspw. US 7,383,077 B2).

Auch ist bekannt, einen Messkopf in seinen Dimensionen größer zu gestalten und die beide Lichtwege (Anregungs- und Detektionslichtweg) getrennt über Linsen / Spiegel und Filter zu trennen. Dies wird u.a. von Shim, M.G. in "Evaluation of fiber optic probes for in-vivo Raman spectroscopy" [Proceedings of SPIE 3257 208 - 217 (1998)] und von Short, M.A. et al. in "Development and preliminary results of an endoscopic Raman probe for potential in vivo diagnosis of lung cancer." [Optics Letters 33, 711-713 (2008)] vorgeschlagen.

Da Anregungsweg und Sammelweg unterschiedliche sowie genau gegensätzliche Filter benötigen, werden gemäß diesem Stand der Technik immer Sonden mit getrenntem Anregungs- und Sammelweg vorgeschlagen, welche Punktsonden sind.

Dieser Aufbau hat den Nachteil, dass er sehr voluminös ist und nicht in kleine Gefäße oder Katheter eingeführt werden kann bzw. der ungefilterte Teil der Fasern nur sehr kurz sein darf, da sonst die Störsignale die gewünschten Raman- Signale überlagern.

Auch kann mit diesen Punktsonden keine Bildgebung für schnelle Übersichtsbilder erfolgen.

Aus der DE 94 14 467 U1 ist bspw. ein Raman- Spektrometer mit einer externen, ein Lichtleiterbündel enthaltenden Messsonde zum Aufsetzen auf die Oberfläche einer Probe und mit einem Laser zur Anregung von Raman- Strahlung in der Probe bekannt, wobei das Laserlicht in eine oder mehrere zentrale Fasern des Lichtleiterbündels eingekoppelt und über ein Fokussierelement auf die Probenoberfläche geführt wird und das von der Probe emittierte Raman- Licht über das Fokussierelement in weitere Fasern des Lichtleiterbündels eingekoppelt und in das Gehäuse des Spektrometers eingeleitet wird.

Das Fokussierelement enthält dabei einen transparenten optischen Körper mit einer ersten planen Oberfläche, die mit einer ebenfalls planen Endoberfläche des Lichtleiterbündels entweder direkt in Kontakt ist oder auf welche die Endoberfläche des Lichtleiterbündels abgebildet wird, mit einer zweiten Oberfläche, die mit der Probenoberfläche in Kontakt gebracht werden kann und mit einer dritten, mantelförmig geschlossenen, verspiegelten oder totalreflektierenden Oberfläche.

Das Fokussierelement ist dabei lösbar auf das Ende des Lichtleiterbündels aufgesteckt und in die zentralen Fasern des Lichtleiterbündels ist ein optischer Filtereinsatz eingesetzt.

Der optische Filtereinsatz enthält zwei transparente Kugeln oder Gradientenindexlinsen (GRIN-Linsen) zur Lichtkollimation, zwischen denen sich eine Filterplatte (Interferenzfilter) befindet, deren Ebene nicht exakt senkrecht auf die Verbindungslinie der Mittelpunkte der beiden Kugeln oder GRIN- Linsen steht.

Dieser Filtereinsatz dient der Unterdrückung von unerwünschter Raman-Streuung des Fasermaterials in die zentralen Fasern. Die leicht geneigte Filterplatte (der leicht geneigte Interferenzfilter) bewirkt, dass Licht des Lasers nicht direkt rückreflektiert wird.

GRIN-Linsen zum Lichtmanagement an sich sind des Weiteren auch aus der Offenbarung von US 2008/119832 A1 bekannt.

Die DE 10 2004 015 946 B3 offenbarteine Vorrichtung (Raman- Sonde) und ein Verfahren zur Erzeugung und zur Detektion eines Raman-Spektrums eines zu untersuchenden Mediums, welche bei vorgegebenen Anforderungen hinsichtlich der stofflichen Analytik (Zahl der Komponenten, Konzentrationen, erforderliche Nachweisgrenzen) eine Erzeugung und Detektion eines Raman- Spektrums ermöglichen. Die Raman- Sonde weist dabei eine optische Faser auf, wobei das erste Ende der optischen Faser über ein Mittel zum Einkoppeln und Auskoppeln von Licht, d. h. über ein geeignetes optisches Kopplungselement sowohl mit einer (Anregungs-) Lichtquelle als auch mit einer Detektionseinheit zur Erfassung eines Raman- Spektrums verbunden ist und über einen freigelegten Teil der optischen Faser Licht in das zu untersuchende Medium ausgekoppelt und Rückstreulicht aus dem zu untersuchenden Medium in die Faser eingekoppelt wird.

Der Nachteil der beiden technischen Lösungen gemäß DE 94 14 467 U1 und DE 10 2004 015 946 B3 besteht darin, dass die Raman- Sonde immer auf den Punkt aufgesetzt bzw. fokussiert werden muss, der gemessen werden soll, so dass die Sonde in ihrer Position geändert werden muss.

Hinzu kommt, dass die Raman- Spektren gemäß DE 94 14 467 U1 und DE 10 2004 015 946 B3 für eine Bildgebung in Form schneller Übersichtsbilder, bspw. im Zusammenhang mit der Untersuchung von biologischem Gewebe, ungeeignet ist.

Aus der Publikation von Kenny Kong et al. "Diagnosis of tumors during tissue-conserving surgery with integrated autofluorescence and Raman scattering microscopy" [Kenny Konga, Christopher J. Rowlandsa, Sandeep Varmab, William Perkinsb, Iain H. Leachc, Alexey A. Koloydenkod, Hywel C. Williamse and Ioan Notinghera: "Diagnosis of tumors during tissue-conserving surgery with integrated autofluorescence and Raman scattering microscopy" PNAS, 09/2013, Vol. 110 no. 38, page 15189 - 15194; Offenbarung der Publikation online unter: (www.pnas.org/cgi/doi/10.1073/ pnas.1311289110)] ist die Kombination beider Techniken (Fluoreszenz- und Raman-Spektroskopie) vermittels eines kombinierten Fluoreszenz- und Raman-Mikroskops bekannt, welche zu einem enormen Zeitvorteil gegenüber den bekannten Untersuchungsmethoden führt.

Die Fluoreszenzspektroskopie wird bei dieser kombinierten Methode genutzt, um das erfasste Bild des biologischen Gewebes in s.g. Cluster aufzuteilen. Dies wird durch die Anregung der Autofluoreszenz bei zwei verschiedenen Wellenlängen realisiert. Die Intensitäten der auftretenden Autofluoreszenz werden dabei zueinander ins Verhältnis gesetzt und das Bild so in viele Cluster mit gleichen Intensitätsverhältnissen geteilt. Innerhalb eines solchen Abschnittes, so postuliert Kong et al., ändert sich der Gewebetyp nicht. D.h. mit der Aufnahme und Klassifikation eines einzelnen (oder mehreren) Raman- Spektren innerhalb eines solchen Clusters kann der vorliegende Gewebecluster sehr spezifisch klassifiziert werden und die Aufnahmedauer eines gesamten und gleichzeitig sehr spezifischen Bildes extrem reduziert werden.

Der Nachteil dieser technischen Lösung besteht darin, dass ein Mikroskop mit Objektiv verwendet werden muss, um das biologische Gewebe *in vitro* zu charakterisieren, da die faserbasierte Aufnahme von Raman- Spektren Filter auf einer Sondenspitze erfordert.

Eine endoskopische Untersuchung von biologischem Gewebe *in vivo* (bspw. im Lungen, Prostata- oder Darmbereich eines Patienten) ist mit dieser technischen Lösung nicht möglich.

US 2012/0075627 A1 offenbart eine Vorrichtung zur Ramanspektroskopischen Untersuchung von fluiden Proben in einer Fluidplattform. Dabei wird Raman-Anregungslicht über eine Faser in einen Messkopf eingestrahlt, um damit die Probe zu beaufschlagen. Das davon emitierte Raman-Signallicht gelangt wieder in denselben Messkopf und wird dann durch einen Strahlteiler in eine andere Faser eingekoppelt.

Mit dieser Vorrichtung werden fluide Proben untersucht, welche die Mikrofluid-Plattform durchströmen. Feste Proben oder in situ-Endoskopieuntersuchungen an Proben sind mit dieser Vorrichtung nicht möglich.

US 2013/0034498 A1 beschreibt einen optischen Kubus, der für verschiedene spektroskopische und mikroskopische Untersuchungsmethoden verwendet werden kann. Je nach Anwendung (bspw. Raman, SERS, Fluoreszenz, Rastermikroskopie oder DNA-Assays) können die Innen- und/oder Außenseiten des optischen Kubus funktionalisiert beschichtet und/oder belegt werden (bspw. DNA-Material).

Ein Messkopf, bspw. für endoskopische in-situ-Untersuchungen ist bei dieser technischen Lösung nicht vorgesehen.

US 2001/0052979 A1 offenbart eine Vorrichtung mit der man simultan fluoreszenz- und Raman-spektroskopische sowie - mikroskopische Untersuchungen durchführen kann. Dabei wird ein Oberflächenbild der Probe erzeugt, das man bei Bedarf punktuell weitergehend untersuchen kann. Diese Vorrichtung umfasst u.a. auch einen optischen Kubus zur polarisationsabhängigen Untersuchung von Proben, welcher in die Optik der Vorrichtung integriert ist. Optische Fasern zur Lichtleitung zu und von der Probe weg, die bspw. endoskopische in-situ-Untersuchungen erfordern, sind bei dieser Vorrichtung nicht vorgesehen.

US 2009/021724 A1 offenbart eine Vorrichtung für die nichtinvasive Beurteilung der morphologischen Details von Gewebearealen vermittels Kohärenztomographie, zur strukturellen Evaluierung für die Zuordnung der gesammelten Ramansignalstrahlung im morphologischen Abbild des Gewebeareals und vermittels der Analyse der biochemischen Zusammensetzung des Gewebeareals durch die Raman-Spektroskopie.

Die US 2009/0021724 A1 offenbart eine Vorrichtung für die nichtinvasive Beurteilung der morphologischen Details von Gewebearealen (vermittels Kohärenztomographie zum Zweck der strukturellen Evaluierung zur Zuordnung der gesammelten Ramansignalstrahlung im morphologischen Abbild des Gewebeareals und für die Analyse der biochemischen Zusammensetzung des Gewebeareals vermittels der Raman-Spektroskopie.

Dazu wird eine tomographische Bildgebungstechnik im Mikrometermaßstab mit axialer und lateraler Auflösung eines Gewebeareals durch Kohärenztomographie mit Breitbandlicht verwendet.

Dabei funktioniert die Kohärenztomographie (OCT) als eine Art optischer Version von Ultraschall. Während bei den Ultraschallbildern durch einen Wandler die emittierten Ultraschallimpulse mit dem detektierten Schallecho des Gewebes korreliert werden, werden bei den OCT-Bildern Referenz- und Probenbreitbandlicht auf ein Gewebe gesendet und das reflektierte Referenzlicht und das an dem Gewebe stark gestreute Reflektionslicht vermittels eines Interferometers korrelieren, wobei auf Grund der extrem hohen Lichtgeschwindigkeit die Niederkohärenz-Interferometrie anstatt der Impuls-Echo-Laufzeitmessungen zum Einsatz kommen, wobei bspw. ein Niederkohärenz-Michelson-Interferometer verwendet wird.

Die nichtinvasive Beurteilung der morphologischen Details von Gewebearealen beruht dabei auf dem Abrastern einer Probe mit zwei galvanischen Spiegeln, wie in Fig. 1 der US 2009/0021724 A1 dargestellt.

Das zentrale Element sind somit die beiden galvanischen Spiegel, wobei der Lichtstrahl über diese Spiegel und über die Linse OL auf die Probe abgebildet wird, so dass ein punktweises Abrastern der Probe erfolgen kann.

Der Nachteil dieser technischen Lösung gemäß der Offenbarung von US 2009/0021724 A1 besteht darin, dass sich diese galvanischen Spiegel nicht wesentlich verkleinern lassen, da ein gewisses Drehmoment des Elektromotors notwendig ist, um den Spiegel zu bewegen.

Die kleinsten, derzeit bekannten Motoren, welche Spiegel-ähnliche Aufsätze haben, besitzen einen Durchmesser von 0,6 mm (Biomed Opt Express. 2015 Dec 1; 6(12): 5021-5032.Published online 2015 Nov 23. doi: 10.1364/BOE.6.005021; Heartbeat OCT: in vivo intravascular megahertz-optical coherence tomography; Tianshi Wang et al.). Diese technischen Lösungen erlauben keine gezielte vor und Rückwärtsbewegung, die Motoren haben eine Länge von etwa 3 mm. Um die Scanbewegung mit ihnen gemäß US 2009/0021724 A1 zu erzeugen, müssen beide Spiegel senkrecht zueinander stehen. Somit ist einer der beiden Spiegel für die x-Richtung und der andere der beiden Spiegel für die y-Richtung notwendig. Wollte man damit eine miniaturisierte Sonde bauen, so wäre der Bauraum (durch geschicktes positionieren der Motoren mit mikropositionierten Spiegeln) somit mindestens 3 mm im Durchmesser + Abmessungen des Spiegels (z.B. 1 mm). Mal abgesehen von starken Positionierungsfehlern dieser miniaturisierten Spiegel und den notwendigen Kabeln müssten noch gewisse optische Elemente mit in den Kopf der Anordnung verbaut werden - Filter, Linsen etc. um diesen Kopf zum Funktionieren zu bringen. Damit lässt sich aber der extrem miniaturisierte Bauraum von 4 mm Durchmesser für eine endoskopisch einzuführende Sonde (bspw. zum Einführen in den Darm eines Menschen) nicht einhalten.

Weiterhin hat die technische Lösung gemäß der Offenbarung von US 2009/0021724 A1 den Nachteil, dass immer noch Kabel für die Treiberspannung der Motoren in diesem Kopf notwendig sind. Das ist problematisch, da
a) zum Anschließen gesonderte elektrische Anschlüsse zum Betreiben der beiden Motoren notwendig sind sowie
b) eine extrem gute Isolation gegen Flüssigkeit vorhanden sein muss, um den Patienten durch die teils sehr hohen Spannungen nicht zu gefährden. Eine bloße Miniaturisierung der technischen Lösung gemäß der Offenbarung von US 2009/0021724 A1 ist somit extrem kritisch für das Patientenwohl.

US 2009/231578 A1 offenbart ein Mehrkanal-Optik-Spektroskopie-System für die Raman-Spektroskopie, das eine Vielzahl von optischen Kanälen, und eine integriertes Optik-Modul aufweist, bei dem das Optikmodul
- einen dichroitischen Strahlteiler, der bspw. zwischen einem ersten transparenten Dreiecksblock und einen zweiten Dreiecks-Block angeordnet ist,
- ein Laserlinienfilter,
- ein Langpasskantenfilter ,
- eine probenseitige optische Faser an einem Ende eines integrierten Optik-Modul und am anderen Ende zur Verbindung mit einer optischen Mehrkanal- Sonde ist, so dass Laserlicht zur Probe zuführbar und Licht von der Probe zu dem dichroitischen Strahlteiler leitbar ist,
- eine Lichtquellen-seitige optischen verbunden mit einer Lichtquelle an einem Ende und mit dem integrierten Optikmodul an dem anderen, so dass Licht von der Lichtquelle zuführbar ist durch den Laserlinienfilter in Richtung auf und durch den dichroitischen Strahlteiler geleitet wird, um die Probe zu beleuchten, wobei eine Kollimatorlinse (bspw. eine Grin-Linse) zwischen der Laser-seitigen optischen Faser und dem Laserlinienfilter des Optikmoduls in den Strahlengang vom Laser in Richtung Probe einfügt sein,
- ein Spektrometer- seitigen Lichtleiter, um Licht von der Probe, das von der optischen Faser aufgefangen wird und von dem dichroitischen Strahlteiler zu dem Spektrometer reflektiert wird, zu liefern;
- ein optisches Spektrometer, um gleichzeitig optische Signale von mehr als einer Spektrometer- seitigen optischen Faser zu empfangen und getrennt zu messen und
- eine Laserlichtquelle, um gleichzeitig Licht in mehrere optische Kanäle einzuleiten, umfasst.

Sowohl die US 2009/021724 A1 als auch die US 2009/231578 A1 haben den Nachteil, dass sie keine biochemisch- funktionelle Auswahl von Teilen in Gewebesarealen (bspw. einem Tumorgewebe in einem umgebenden gesunden Gewebe) zum Zweck der anschließenden gezielten Positionierung von Raman-Anregungsstrahlung zur speziellen Charakteriesierung der Tumorzellen (bspw. nach Tumor-Zelltypen) bei der *in vivo-* Endoskopie ermöglichen.

Der Erfindung liegt die Aufgabe zugrunde, eine Vorrichtung mit einer Raman- Sonde sowie ein Verfahren unter Verwendung dieser Vorrichtung anzugeben, welche die Nachteile des Standes der Technik vermeiden, insbesondere eine schnelle endoskopische Untersuchung von biologischem Gewebe *in vivo* ermöglichen, wobei eine Klassifizierung nach Gewebetyp erfolgt.

Gelöst wird diese Aufgabe erfindungsgemäß durch eine Vorrichtung zur Erzeugung und zur Detektion von Red-Flag-Strahlung sowie eines Raman- Spektrums gemäß dem ersten Patentanspruch sowie einem Verfahren gemäß dem 8. -Patentanspruch. Vorteilhafte Ausgestaltungen der Erfindung sind in den nachgeordneten abhängigen Ansprüchen angegeben.

Das Konzept besteht darin, dass durch die endoskopische Bereitstellung eines schnellen Red-Flag- Strahlungsübersichtsbildes (bspw. eines schnellen Fluoreszenzübersichtsbildes) eine Unterteilung eines zu untersuchenden Ausschnitts eines biologischen Gewebes *in vivo* erfolgt und in Kombination mit einer anschließenden spezifischen Charakterisierung mittels Raman- Spektroskopie *in vivo* in den entsprechend unterteilten, ausgewählten Regionen des im Übersichtsbild dargestellten Gewebes eine Klassifizierung nach Gewebetyp erfolgt, ohne eine Entnahme von biologischem Gewebe aus inneren oder äußeren Körperregionen vornehmen und ohne die Position der Sonde nachstellen zu müssen.

Dazu umfasst die Vorrichtung zur Erzeugung und zur Detektion von Red-Flag- Strahlung sowie eines Raman- Spektrums eine Laserlichtquelle zur Generierung von Strahlung in einer biologischen Gewebeprobe und eine, ein Bündel von Lichtleitern (zentrale Faser) und eine externe Faser enthaltende Messsonde zum Aufsetzen auf die Oberfläche der biologischen Gewebeprobe, wobei diese Gewebeprobe durch die über das Bündel von Lichtleitern führbare Strahlung der Laserlichtquelle (Red- Flag- Anregungsstrahlung) abrasterbar ist, um ein Red-Flag- Übersichtsbild der Gewebeprobe mittels der gleichen oder einer anderen externen Faser zu erzeugen, welches in das Gehäuse einer Detektions- und Steuereinheit auf einen Detektor für Red- Flag-Strahlung leitbar ist.

Dabei ist anschließend Laserlicht (Raman- Anregungsstrahlung) in eine (oder mehrere) zentrale Faser(n) des Bündels vermittels der Detektions- und Steuereinheit an Hand des Red-Flag- Übersichtsbildes gezielt, adressierbar einkoppelbar und über ein Fokussierelement auf die Oberfläche der biologischen Gewebeprobe führbar, so dass an jedem Punkt des Red-Flag- Übersichtsbildes gezielt adressierbar Raman-Strahlung für die Aufnahme von Ramanspektren erzeugbar ist, ohne die Messsonde bewegen zu müssen.

Die von der Gewebeprobe emittierte, von der Messsonde erfasste Raman- Strahlung ist dabei über die externe Faser der Messsonde in das Gehäuse der Detektionseinheit in ein einem Raman- Spektrometer leitbar.

Wesentlich dabei ist, dass das Fokussierelement am Kopf der Messsonde in Form einer transparenten optischen Linse (für die Anregungsstrahlung für die Red-Flag- und die Raman- Strahlung) und einer unmittelbar daneben angeordneten transparenten optischen Linse (für die Signalstrahlung in Form der Red-Flag- und der Raman- Strahlung) angeordnet ist. Vermittels dieser beiden Linsen ist das Laserlicht der Fasern des Lichtleiterbündels als Anregungsstrahlung kollimiert einkoppelbar und die Signalstrahlung in die bildgebenden Fasern auskoppelbar.

Wesentlich ist dabei weiterhin, dass der Linse, welche das Anregungslicht für die Raman- Strahlung kollimiert, ein transparenter optischer Kubus mit einer anschließenden transparenten dritten optischen Linse nachgeordnet ist, so dass der Kubus direkt zwischen der Linse der Raman- Anregungsstrahlung und dieser dritten Linse positioniert ist, wobei in dem Kubus eine Filterschicht, eine Absorber, ein Spiegel und ein Filter in der Art eingebracht sind, dass durch die Filterschicht der in den Fasern (der Raman- Anregungsstrahlung) entstandene Untergrund auf den Absorber lenkbar ist (Adsorption von Strahlung größer als 800 nm aus der Raman- Anregungsstrahlung) und die "bereinigte" Raman- Anregungsstrahlung durch die dritte Linse auf die Oberfläche der biologischen Gewebeprobe führbar ist.

Die durch die Probe generierte Raman- Signalstrahlung ist vermittels der dritten Linse sammel- und kollimierbar.

Wichtig dabei ist, dass die Raman- Anregungs- und die Raman-Signalstrahlung durch den Filter im Kubus trennbar ist, so dass die durch den Filter (in Form eines Notchs oder Bandpass) abgetrennte Raman-Signalstrahlung im Kubus durch den Spiegel umlenkbar und über Linse (für die Raman- Signalstrahlung) in die externe bildgebende Faser einkoppelbar ist. Dadurch erfolgt durch den Filter die "Bereinigung" des Raman- Signals von der Anregungsstrahlung, wobei die bereinigte Raman- Signalstrahlung in das Gehäuse der Detektionseinheit mit Raman- Spektrometer mündet.

Die Detektions- und Steuereinheit dient dabei der Detektion und Auswertung des schnellen Red-Flag- Übersichtsbildes eines biologischen Gewebes (bspw. in Form eines schnellen Fluoreszenzübersichtsbildes), der Bildunterteilung und der Auswahl der Gebiete des unterteilten Red-Flag- Übersichtsbildes zur Steuerung für die anschließende spezifische Charakterisierung des Gewebeausschnittes vermittels Raman- Spektroskopie im Sinne der Publikation "Diagnosis of tumors during tissue-conserving surgery with integrated autofluorescence and Raman scattering microscopy" von Konga et. al [Kenny Konga, Christopher J. Rowlandsa, Sandeep Varmab, William Perkinsb, Iain H. Leachc, Alexey A. Koloydenkod, Hywel C. Williamse and Ioan Notinghera: "Diagnosis of tumors during tissue-conserving surgery with integrated autofluorescence and Raman scattering microscopy" PNAS, 09/2013, Vol. 110 no. 38, page 15189 - 15194; Offenbarung der Publikation online unter: www.pnas.org/cgi/doi/10.1073/pnas.1311289110).

Dabei ist Laserlicht in Form der Raman- Anregungsstrahlung gezielt und adressierbar in eine (oder mehrere) zentrale Faser(n) des Bündels einkoppelbar und über den Kubus mit dem Fokussierelement auf die Oberfläche der biologischen Gewebeprobe führbar.

Dabei sind die drei verwendeten Linsen als Gradientenindexlinsen ausgeführt, wobei das Bündel von Lichtleitern sowie die externe Faser zu einem Faserkombinationsbündel zusammengefasst und gemeinsam mit der Messsonde von einer im Wesentlichen hohlzylindrischen Umhüllung umgeben werden können (bspw. ein Kanal eines Endoskops). Dabei ist die Stirnfläche der dritten Linse nicht umhüllt und der Innendurchmesser des Hohlzylinders stimmt im Wesentlichen mit dem Außendurchmesser des Faserkombinationsbündels und der Messsonde überein.

Das Faserkombinationsbündel kann bis zu 10 Metern lang sein und einen Außendurchmesser von bis zu 10 mm aufweisen.

Das Faserkombinationsbündel aus dem Bündel von Lichtleitern mit zentraler Faser und der externen Faser besteht aus Quarzglasfasern, wobei das Bündel von Lichtleitern die Red-Flag- Anregungsstrahlung sowie die Red-Flag- Strahlung transportiert und die zentrale bildgebende Faser (Imaging- Faser) umfasst, welche die Raman- Anregungsstrahlung transportiert. Die externe Faser ist dabei eine Multimode-Faser mit einem Durchmesser von bspw. 100 µm, welche sämtlich Raman-Signalstrahlung transportiert.

Die bildgebende Faser kann dabei mit vielen lichtleitenden Kernen umgeben sein oder als Faserbündel aus vielen einzelnen zu dem Bündel zusammengesetzten Fasern bestehen.

Der Adsorper adsorbiert sämtliche Strahlung über 800 nm.

Der Filter (bspw. ein Notch oder Bandpass) "bereinigt" des Raman-Signals, die größer als 800 nm ist, von der Anregungsstrahlung.

Die verwendet Laserlichtquelle ist bspw. ein Nd-YAG-Laser, ein He-Ne-Laser oder ein Halbleiterlaser.

Die von diesem Laser vermittels Anregungsstrahlung erzeugte Red-Flag-Strahlung ist eine Autofluoreszenz- Strahlung, eine Zwei- oder Dreiphotonenfluoreszenz- Strahlung, kohärente Anti-Stokes-Raman-Strahlung oder stimulierte Raman- Streuungsstrahlung.

Die Detektions- und Steuereinheit ist eine geeignete, an sich bekannte Scanneroptik (bspw. Laserscanningmikroskop), vermittels derer die Red-Flag- Strahlung detektierbar ist, welche datenleitend mit der Software eines Computers in Verbindung steht.

Der Steuerteil der Detektions- und Steuereinheit übernimmt dabei die von dem Detektionsteil der Detektions- und Steuereinheit erzeugten, vermittels der Software des Computers bearbeiteten Signale (ausgewertetes Red- Flag- Übersichtsbild) und dient der Steuerung der Raman- Anregungsstrahlung, in dem die Position der von der Laserlichtquelle in das Bündel von Lichtleitfasern einkoppelbare Raman-Anregungs-strahlung gezielt, adressierbar auf Punkte des im Red- Flag-Übersichtsbild dargestellten, zu untersuchenden biologischen Gewebes richtbar ist, welche den zuvor gezielt ausgewählten Punkten / Spots im Red- Flag- Übersichtsbild oder dem entsprechenden Clusterbild (wie Kenny Kong et al.) entsprechen.

Bei dem Verfahren zur Erzeugung und zur Detektion von Red-Flag-Strahlung und eines Raman- Spektrums unter Verwendung der erfindungsgemäßen Vorrichtung wird mittels der Laserlichtquelle eine Anregungsstrahlung über die bildgebende Faser durch die Linse für die Anregungsstrahlung, durch die Filterschicht des Kubus und dritte, dem Kubus nachgeordnete Linse hindurch auf die Oberfläche der biologischen Gewebeprobe geführt und vergrößert oder verkleiner abgebildet.

Die von der Probe emittierte Red-Flag- Signalstrahlung wird dann von der dritten, dem Kubus nachgeordnete Linse über die Filterschicht des Kubus, durch den Filter hindurch über den Spiegel im Kubus durch die Linse für die Faser hindurch in die Bildleitfaser geführt.

Die Signalstrahlung in Form der Red-Flag- Strahlung wird in die Detektionseinheit geleitet, wobei auf Grundlage der ausgewerteten Signale der Detektionseinheit über die Auswerte- und Steuereinheit die Raman- Anregungsstrahlung von der Laserlichtquelle gezielt adressierbar, einzelne in eine zentrale Faser des Bündels eingeleitet wird.

Die Raman- Anregungsstrahlung wird dabei ebenfalls über die bildgebende Faser, durch die Linse für die Anregungsstrahlung, durch die Filterschicht des Kubus und die dritte, dem Kubus nachgeordnete Linse hindurch auf die Oberfläche der biologischen Gewebeprobe geführt.

Die von der Probe emittierte Raman- Signalstrahlung wird ebenfalls von der dritten, dem Kubus nachgeordnete Linse über die Filterschicht des Kubus, durch den Filter im Kubus hindurch über den Spiegel im Kubus durch die Linse für die Signalstrahlung hindurch in die externe Faser (Multimodefaser) geführt.

Die Signalstrahlung in Form Raman- Strahlung wird dabei in das Raman- Spektrometer weitergeleitet, wobei aus dem detektierten Raman- Spektrum des gezielt ausgewählt untersuchten Ausschnitts aus der Oberfläche der biologischen Gewebeprobe Strukturparameter des Gewebes ermittelt werden, die Rückschlüsse auf die Eigenschaften des zu charakterisierenden Gewebeausschnitts liefern.

Durch den Einsatz der erfindungsgemäßen kombinierten Vorrichtung zur Erzeugung und zur Detektion von Red-Flag- Strahlung und eines Raman- Spektrums ist es möglich, eine endoskopische Untersuchung von biologischem Gewebe *in vivo* durchzuführen, wobei die Messdauer gering ist, da vermittels der angewendeten "Red-Flag"- Technik (bspw. Bereitstellung eines Fluoreszenzübersichtsbildes für die Gewebeausschnittunterteilung) eine Bildunterteilung mit gezielter Bereichsauswahl für eine anschließende spezifische Charakterisierung des biologischen Gewebes im ausgewählten Bereich mittels Raman-Spektroskopie (bspw. Tumorgewebe, kein Tumorgewebe) möglich ist.

Durch die kleine Bauform sind die bildgebende Faser, die Bildleitfaser und der Faserkopf / die Messsonde mit erfindungsgemäß ausgestaltetem Kubus in eine Umhüllung, insbesondere einen Kanal eines Endoskops integrierbar, so dass eine Klassifizierung nach dem Typ des biologischen Gewebes erfolgen kann, ohne eine Entnahme von biologischem Gewebe aus inneren Körperregionen vornehmen zu müssen.

Eine Erweiterung des Konzeptes der Vorrichtung mit einer Ramansonde und des Verfahrens unter Verwendung dieser Vorrichtung auf Prozesse allgemein, welche einen intrinsischen Untergrund in optischen Fasern oder Kombinationen von optischen Fasern generieren, liegt ebenfalls im Rahmen der Erfindung. Anwendungsbeispiele dafür sind:
- Fluoreszenz
- Zweiphotonenfluoreszenz
- Dreiphotonenfluoreszenz
- Second-Harmonic-Generation
- Raman
- CARS
- OCT

Die Erfindung wird nachstehend an Hand der Ausführungsbeispiel und der Figuren näher erläutert. Dabei zeigen:
- Fig. 1:: eine schematische Übersichtsdarstellung einer Ausführungsform der Vorrichtung zur Erzeugung und zur Detektion von Red-Flag-Strahlung und eines Raman- Spektrums,
- Fig. 2:: eine schematische Darstellung der Messsonde gemäß Fig. 1,
- Fig. 3:: eine weitere schematische Übersichtsdarstellung,
- Fig. 4:: eine schematische Darstellung der Linearanordnung der "19 MM Fasern" gemäß Fig. 3 und
- Fig. 5:: eine schematische Darstellung der über den äußeren Umfang der Anregungsfaser verteilten "19 MM Fasern" gemäß Fig. 3.

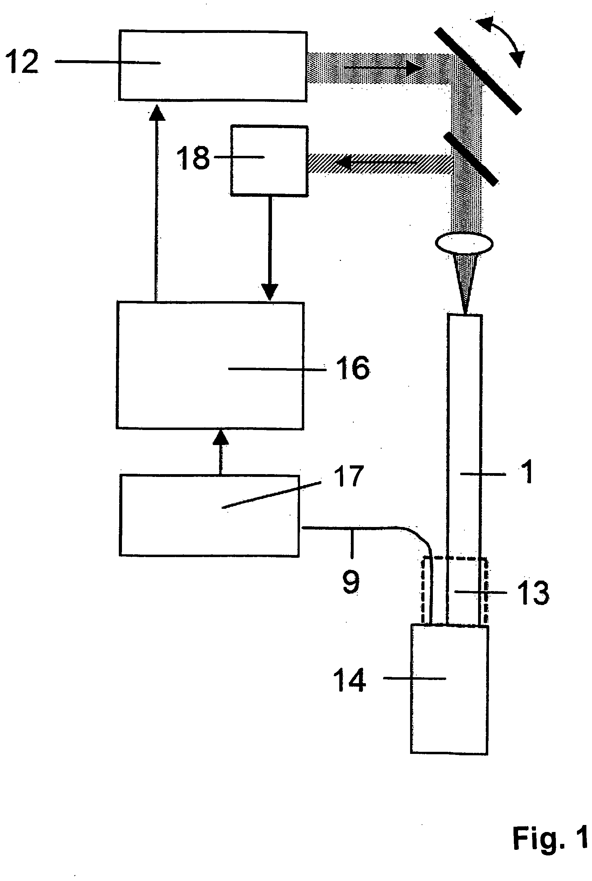

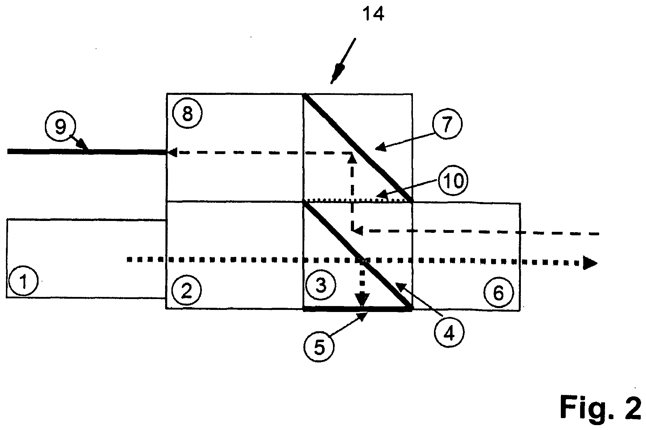

Die in Fig. 1 schematisch dargestellte Vorrichtung zur Erzeugung und zur Detektion von Red-Flag- Strahlung und eines Raman- Spektrums umfasst eine Laserlichtquelle (12) zur Anregung von Strahlung in einer biologischen Gewebeprobe und eine externe, ein Bündel von Lichtleitern mit mindestens einer Faser (1) enthaltende Messsonde (14) zum Aufsetzen auf die Oberfläche der biologischen Gewebeprobe, wobei Laserlicht in eine oder mehrere Fasern (1) des Bündels von Lichtleitern einkoppelbar und über ein Fokussierelement auf die Oberfläche der biologischen Gewebeprobe führbar ist und von der Gewebeprobe emittiertes Licht über das Fokussierelement in eine weitere Faser (9) einkoppelbar und in das Gehäuse einer Detektionseinheit (16) mit einem Raman- Spektrometer leitbar ist, wobei dass das Fokussierelement am Kopf der Messsonde (14), wie in Fig. 2 dargestellt, in Form einer transparenten optischen Linse (2) und einer unmittelbar daneben angeordneten transparenten optischen Linse (8) angeordnet ist, vermittels derer das Laserlicht der Faser (1) als Raman-Anregungsstrahlung kollimiert einkoppelbar und die Raman-Signalstrahlung in die externe Faser (9) auskoppelbar ist.

Dabei ist der Linse (2), wie in Fig. 2 dargestellt, ein transparenter optischer Kubus (3) mit anschließender transparenter optischer Linse (6) nachgeordnet, so dass der Kubus (3) direkt zwischen der Linse (2) und der Linse (6) positioniert ist, wobei in dem Kubus (3) eine Filterschicht (4), eine Absorber (5), ein Spiegel (7) und ein Filter (10) in der Art eingebracht sind, dass durch die Filterschicht (4) der in den Fasern (1) entstandene Raman- Untergrund auf den Absorber (5) lenkbar und Raman- Anregungsstrahlung durch eine Linse (6) auf die Oberfläche der biologischen Gewebeprobe führbar ist.

Die durch die Probe generierte Raman- Signalstrahlung ist durch die Linse (6) sammel- und kollimierbar, wobei die Raman- Anregungs- und die Raman- Signalstrahlung durch den Filter (10) im Kubus (3) trennbar ist, so dass die durch den Filter (10) abgetrennte Raman- Signalstrahlung im Kubus (3) durch den Spiegel (7) umlenkbar und über die Linse (8) in eine externe Faser (9) koppelbar ist.

Die in eine externe Faser (9) eingekoppelte Signalstrahlung ist in das Gehäuse der Detektions- und Steuereinheit (16) mit Raman-Spektrometer (17) einleitbar.

Die in der Fig. 2 dargestellte Linse (2), die Linse (6) und die Linse (8) sind als Gradientenindexlinsen ausgeführt.

Das Bündel von Lichtleitern, welches mindestens eine Faser (1) enthält sowie die externe Faser (9) sind zu einem Faserkombinationsbündel (13) zusammengefasst, welches gemeinsam mit der Messsonde (14) von einer (in der Figur nicht dargestellten) im Wesentlichen hohlzylindrischen Umhüllung umgeben sein, wobei die Stirnfläche der Linse (6) nicht umhüllt ist und der Innendurchmesser des Hohlzylinders im Wesentlichen mit dem Außendurchmesser des Faserkombinationsbündels (13) / der Messsonde (14) übereinstimmt. Die nicht in der Fig. 2 dargestellte Umhüllung kann bspw. ein Kanal eines Endoskops sein. Das Faserkombinationsbündel (13) kann bis zu 10 Metern lang sein, einen Außendurchmesser von bis zu 10 mm aufweisen, aus Quarzglasfasern bestehen.

Die Faser (1) ist eine bildgebende Faser (Imaging- Faser).

Die externe Faser (9) ist eine Multimode-Faser mit einem Durchmesser von bspw. 100 µm.

Der Adsorber (5) adsorbiert sämtliche Strahlung über 800 nm.

Der Filter (10) kann bspw. ein Bandpass zum "Bereinigen" des Raman-Signals von 785 nm- Anregungsstrahlung sein.

Die Laserlichtquelle kann bspw. ein Nd-YAG-Laser, ein He-Ne-Laser oder ein Halbleiterlaser sein.

Die Red-Flag- Strahlung, welche bei der bestimmungsgemäßen Verwendung der Vorrichtung eingesetzt wird, kann bspw. eine Autofluoreszenz- Strahlung, Zwei- oder Dreiphotonenfluoreszenz-Strahlung (SHG oder THG), kohärente Anti-Stokes-Raman-Strahlung (CARS) oder stimulierte Raman- Streuungsstrahlung (SRS) sein.

Der von der Detektions- und Steuereinheit umfasste Detektor (18) für die der Red-Flag- Signalstrahlung kann als photo-optischer Detektor, ausgeführt sein, vermittels derer die Red-Flag- Strahlung als Red- Flag-Übersichtsbild detektierbar ist.

Die Vorrichtung weist, wie in Fig. 1 dargestellt, eine Detektions- und Steuereinheit (16) zur Auswertung des von dem Detektor (18) ermittelten Red- Flag- Übersichtsbild auf, wobei durch die Detektions- und Steuereinheit (16) die Steuerung der Raman- Anregungsstrahlung gezielt adressierbar, einzelne in eine Faser (1) des Bündels von Lichtleitern (13) auf Grundlage der ausgewerteten Signale generierbar ist.

Bei dem Verfahren zur Erzeugung und zur Detektion von Red-Flag-Strahlung und eines Raman- Spektrums wird unter Verwendung einer erfindungsgemäßen Vorrichtung mittels der Laserlichtquelle (12) eine Raman- Anregungsstrahlung über die Faser (1), durch die Linse (2), die Filterschicht (4) des Kubus (3) und die Linse (6) hindurch auf die Oberfläche der biologischen Gewebeprobe geführt.

Die durch die Raman- Anregungsstrahlung von der Probe emittierte Raman- Signalstrahlung wird von der Linse (6) über die Filterschicht (4) des Kubus (3), durch den Filter (10) hindurch über den Spiegel (7) durch die Linse (8) hindurch in die externe Faser (9) geführt und in das Raman-Spektrometer (17) zur Aufnahme des Raman- Spektrums geleitet.

Dabei wird die Raman- Anregungsstrahlung auf Grundlage zuvor vom Detektor (18) aufgenommenen und vermittels der Software und des Computers der Detektions- und Steuereinheit (16) ausgewerteten Signale des Red-Flag- Übersichtsbildes des zu untersuchenden biologischen Gewebes über die Detektions- und Steuereinheit (16) von der Laserlichtquelle (12) gezielt adressierbar in eine zentrale Faser (1) des Bündels von Lichtleitern eingeleitet.

Auf Grund der Auswertung der Detektions- und Steuereinheit (16) liegt dabei ein Red-Flag- Übersichtsbild über das gesamte zu untersuchende Gewebe vor. In diesem Übersichtsgebiet werden gezielt Cluster mit gleichen Signalintensitätsverhältnissen ermittelt, die als Adressierungsorte für die Raman- Anregungsstrahlung dienen, wobei von jedem Cluster ein Ramanspektrum im Sinne der Publikation "Diagnosis of tumors during tissue-conserving surgery with integrated autofluorescence and Raman scattering microscopy" von Konga et. al [Kenny Konga, Christopher J. Rowlandsa, Sandeep Varmab, William Perkinsb, Iain H. Leachc, Alexey A. Koloydenkod, Hywel C. Williamse and Ioan Notinghera: "Diagnosis of tumors during tissue-conserving surgery with integrated autofluorescence and Raman scattering microscopy" PNAS, 09/2013, Vol. 110 no. 38, page 15189 - 15194; Offenbarung der Publikation online unter: (www.nnas.org/cgi/doi/10.1073/pnas.1311289110) aufzunehmen ist.

Die Raman- Anregungsstrahlung wird dabei gezielt in die Faser (1) eingekoppelt, welche als bildgebende Faser dem adressierten, vorgegebenen Zielgebiet zugeordnet ist (siehe Fig. 2).

Über die Faser (1), durch die Linse (2), die Filterschicht (4) des Kubus (3) und die Linse (6) hindurch wird dabei die Raman-Anregungsstrahlung auf die Oberfläche der biologischen Gewebeprobe geführt und die von der Probe emittierte Raman- Signalstrahlung wird dann von der Linse (6) über die Filterschicht (4) des Kubus (3), durch den Filter (10) hindurch über den Spiegel (7) durch die Linse (8) hindurch in die externe Faser (9) geführt.

Mit Hilfe der Faser (9) wird dann die Signalstrahlung in Form Raman-Anregungsstrahlung in das Raman- Spektrometer (17) geleitet.

Durch die Filterschicht (4) und den Adsorber (5) im Kubus (3) wird dabei die Raman- Anregungsstrahlung auf den Bereich unter 800 nm "bereinigt".

Vermittels des Filters (10) im Kubus (3) wird ermöglicht, dass die Raman- Signalstrahlung im Bereich über 800 nm in die Faser (9) passieren kann.

In dem Raman- Spektrometer (17) werden aus dem detektierten RamanSpektrum des gezielt ausgewählt untersuchten Ausschnitts aus der Oberfläche der biologischen Gewebeprobe Strukturparameter des Gewebes ermittelt, welche Rückschlüsse auf die Eigenschaften des zu charakterisierenden Gewebeausschnitts liefern, in dem das detektierte Spektrum gemäß dem Stand der Technik mit Referenzspektren verglichen wird.

Die zuvor stehende beschriebene Vorrichtung mit einer Ramansonde und das entsprechende Verfahren unter Verwendung dieser ist auch bei Prozessen, welche einen intrinsischen Untergrund in optischen Fasern oder Kombinationen von optischen Fasern generieren, wie bspw.
- Fluoreszenz
- Zweiphotonenfluoreszenz
- Dreiphotonenfluoreszenz
- Second-Harmonic-Generation
- Raman
- CARS
- OCT
anwendbar.

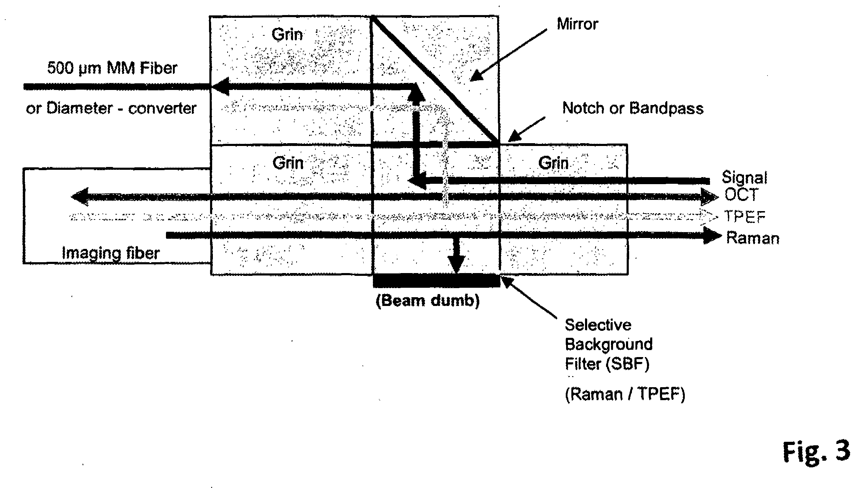

Dazu veranschaulicht die Fig. 3 diese Anwendungsbeispiele schematisch.

Für diese Ausführungsbeispiele ist ein Durchmesser der Sammelfaser (500 mm MM Fiber in Fig. 3) vorgesehen, welcher dem Durchmesser der Anregungsfaser (Imaging Faser in Fig. 3) entspricht, was für praktische Anwendungen, bspw. in der Medizintechnik problematisch sein kann.

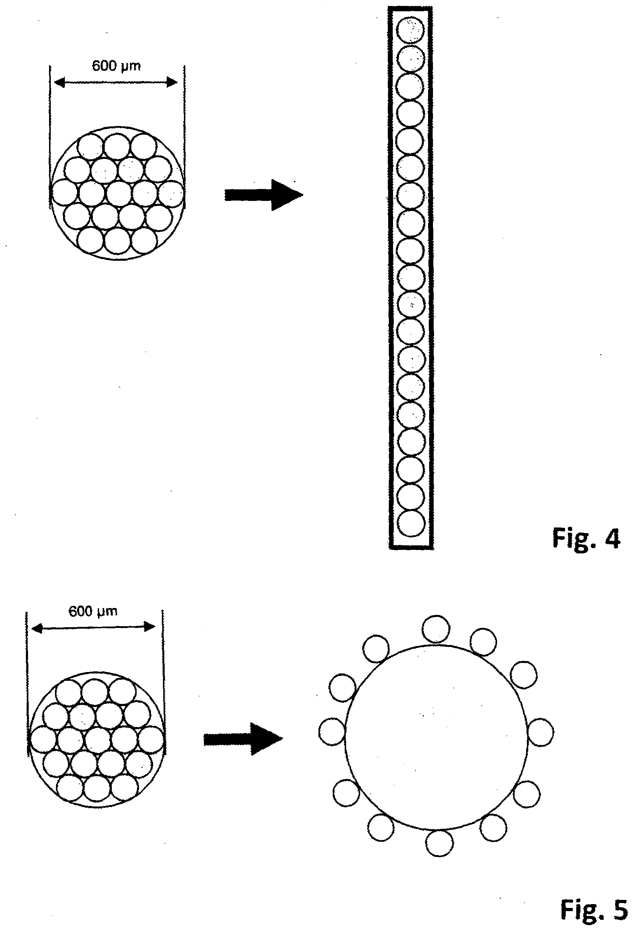

Gelöst wird dieses Problem der gleichen Durchmesser der Faser zum Sammeln sowie wie zum Anregen durch die Verwendung eines Querschnittswandler, bei dem die "19 MM Fasern" nicht (wie üblich) in kompakter Kreisform, sondern zu einer Linie angeordnet sind, wie in Fig. 4 dargestellt.

Die wesentlich kompaktere Ausführung ist nicht nur durch die Linearanordnung der "19 MM Fasern" möglich (siehe Fig. 4), sondern kann auch dadurch erzeugt werden, dass die einzelnen "19 MM Fasern" [deren Summe ihrer Einzeldurchmesser dem Durchmesser der Anregungsfaser (Imaging Faser in Fig. 3) entsprechen] in wesentlichen gleichmäßig um den äußeren Umfang der Anregungsfaser verteilt und von dieser gehaltert sind (siehe dazu Fig. 5).

Die Erfindung ist in den anhängenden Ansprüchen definiert.

### Bezugszeichenliste

- 1 -: Faser (Imaging Faser/bildgebende Faser)
- 12 -: Laserlichtquelle
- 13 -: Faserkombinationsbündel
- 14 -: Messsonde
- 16 -: Detektions- und Steuereinheit
- 17 -: Raman- Spektrometer
- 18 -: Detektor
- 2 -: erste Linse
- 3 -: Kubus
- 4 -: Filterschicht
- 5 -: Absorber
- 6 -: dritte Linse
- 7 -: Spiegel
- 8 -: zweite Linse
- 9 -: externe Faser (Sammelfaser)
- 10 -: Filter

## Patentansprüche

1. Vorrichtung zur Erzeugung und zur Detektion von Red-Flag-Strahlung und eines Raman- Spektrums umfassend eine Laserlichtquelle (12) zur Generierung von Red-Flag- und von Raman- Strahlung in einer biologischen Gewebeprobe, einen Detektor (18) zur Detektion eines Red-Flag- Übersichtsbildes, eine Detektions- und Steuereinheit (16) zur Auswertung des Red- Flag-Übersichtsbildes und zur Steuerung der Raman- Anregungsstrahlung, sowie eine, ein Bündel von Lichtleitern mit mindestens einer Faser (1) enthaltende Messsonde (14) zum Aufsetzen auf die Oberfläche der biologischen Gewebeprobe, wobei die Vorrichtung derart eingerichtet ist, dass das Laserlicht in eine oder mehrere Fasern (1) des Bündels von Lichtleitern einkoppelbar und über ein Fokussierelement auf die Oberfläche der biologischen Gewebeprobe führbar ist und von der Gewebeprobe emittiertes Licht über das Fokussierelement auf den Detektor (18) für Red-Flag-Signalstrahlung und ein Raman-Spektrometer (17) für Raman-Signalstrahlung leitbar ist, wobei das Fokussierelement am Kopf der Messsonde (14) in Form einer ersten transparenten optischen Linse (2) und einer unmittelbar daneben angeordneten zweiten transparenten optischen Linse (8) angeordnet ist, vermittels derer das Laserlicht der Faser (1) als Raman-Anregungsstrahlung kollimiert einkoppelbar und die Raman- Signalstrahlung in die externe Faser (9) auskoppelbar ist, wobei der ersten Linse (2) ein transparenter optischer Kubus (3) mit anschließender transparenter dritter optischer Linse (6) nachgeordnet ist, so dass der Kubus (3) direkt zwischen der ersten Linse (2) und der dritten Linse (6) positioniert ist, wobei in dem Kubus (3) eine Filterschicht (4), ein Absorber (5), ein Spiegel (7) und ein Filter (10) in der Art eingebracht sind, dass durch die Filterschicht (4) der in der Faser (1) entstandene Untergrund auf den Absorber (5) lenkbar und Anregungsstrahlung durch die dritte Linse (6) auf die Oberfläche der biologischen Gewebeprobe führbar ist, durch die Gewebeprobe generierte Raman-Signalstrahlung durch die dritte Linse (6) sammel- und kollimierbar ist, die Raman-Anregungs- und die Raman- Signalstrahlung durch den Filter (10) im Kubus (3) trennbar ist, so dass die durch den Filter (10) abgetrennte Raman-Signalstrahlung im Kubus (3) durch den Spiegel (7) umlenkbar und über die zweite Linse (8) in die externe Faser (9) koppelbar ist, die in das Raman- Spektrometer (17) mündet, wobei die erste Linse (2), die dritte Linse (6) und die zweite Linse (8) als Gradientenindexlinsen ausgeführt sind, und die Red-Flag-Strahlung eine Autofluoreszenz- Strahlung, eine Zwei- oder Dreiphotonenfluoreszenz- Strahlung, eine kohärente Anti-Stokes-Raman-Strahlung oder eine stimulierte Raman- Streuungsstrahlung ist.

2. Vorrichtung gemäß Anspruch 1, **dadurch gekennzeichnet, dass** das Bündel von Lichtleitern mit der Faser (1), die externe Faser (9) und die Messsonde (14) eine im Wesentlichen hohlzylindrische Umhüllung aufweisen, wobei die Stirnfläche der dritten Linse (6) nicht umhüllt ist und der Innendurchmesser des Hohlzylinders im Wesentlichen mit dem Außendurchmesser des Bündels von Lichtleitern zuzüglich der Faser (9) sowie mit dem Außendurchmesser der Messsonde (14) übereinstimmt.

3. Vorrichtung gemäß Anspruch 2, **dadurch gekennzeichnet, dass** die Umhüllung ein Kanal eines Endoskops ist und das Bündel von Lichtleitern mit der mindestens einen Faser (1) sowie die externe Faser (9) zu einem Faserkombinationsbündel (13) zusammengefasst sind.

4. Vorrichtung gemäß Anspruch 1, **dadurch gekennzeichnet, dass** die Laserlichtquelle ein Nd-YAG-Laser, ein He-Ne-Laser oder ein Halbleiterlaser ist.

5. Vorrichtung gemäß Anspruch 1, **dadurch gekennzeichnet, dass** der Detektor (18) ein photo-optischer Detektor ist, vermittels dessen das Red-Flag- Übersichtsbild detektierbar ist.

6. Vorrichtung gemäß einem oder mehrerer der voran stehenden Ansprüche 1 bis 5, **dadurch gekennzeichnet, dass** die Detektions- und Steuereinheit (16) einen Software enthaltenden Computer zur Auswertung der von dem Detektor (18) erzeugten Red-Flag-Übersichtsbildes umfasst, wobei durch die Detektions- und Steuereinheit (16) die Raman- Anregungsstrahlung gezielt adressierbar, einzeln in mindestens eine der Fasern (1) des Bündels von Lichtleitern (13) durch die Strahlung der Laserlichtquelle (12) generierbar ist.

7. Vorrichtung gemäß Anspruch 1, **dadurch gekennzeichnet, dass** der Durchmesser der externen Faser (9) gleich dem Durchmesser der Faser (1) ist, in dem die externe Faser (9) durch einen Querschnittswandler ausgebildet ist, bei dem 19 MM Fasern als Linear- oder Kreisanordnung einzelnen und im wesentlichen gleichmäßig um den äußeren Umfang der Faser (1) verteilt sind und von dieser gehaltert werden, wobei die Summe der Einzeldurchmesser der 19 MM Fasern dem Durchmesser der Faser (1) entspricht.

8. Verfahren zur Erzeugung und zur Detektion von Red-Flag- Strahlung und eines Raman- Spektrums unter Verwendung einer Vorrichtung gemäß einem oder mehrerer der voran stehenden Ansprüche 1 bis 7 bei dem
• mittels der Laserlichtquelle (12) eine Red-Flag- Anregungsstrahlung über das Bündel von Lichtleitern mit mindestens einer Faser (1) auf die Oberfläche einer biologischen Gewebeprobe geführt wird,
• von der Probe emittierte Red-Flag- Signalstrahlung auf den Detektor (18) geführt wird,
• die vom Detektor (18) als Red-Flag- Übersichtsbild erfassten Signale in der Detektions- und Steuereinheit (16) Computergestützt ausgewertet werden wird, wobei auf Grundlage der ausgewerteten vermittels der Detektions- und Steuereinheit (16) die Raman- Anregungsstrahlung von der Laserlichtquelle (12) gezielt adressierbar, einzelne in eine Faser (1) des Bündels von Lichtleitern (13) eingeleitet wird,
• diese Raman- Anregungsstrahlung über die Faser (1), durch die erste Linse (2), die Filterschicht (4) des Kubus (3) und die dritte Linse (6) hindurch auf die Oberfläche der biologischen Gewebeprobe geführt wird,
• von der Probe emittierte Raman- Signalstrahlung von der dritten Linse (6) über die Filterschicht (4) des Kubus (3), durch den Filter (10) hindurch über den Spiegel (7) durch die zweite Linse (8) hindurch in die externe Faser (9) geführt wird,
• die Raman- Signalstrahlung von der Faser (9) in das Raman-Spektrometer (17) geführt wird und
• aus dem im Raman- Spektrometer (17) detektierten RamanSpektrum des gezielt ausgewählt untersuchten Ausschnitts aus der Oberfläche der biologischen Gewebeprobe Strukturparameter des Gewebes ermittelt werden, die Rückschlüsse auf die Eigenschaften des zu charakterisierenden Gewebeausschnitts liefern.

9. Verfahren gemäß Anspruch 8, **dadurch gekennzeichnet, dass** vermittels der Filterschicht (4) und des Adsorbers (5) im Kubus (3) eine bereinigte Raman- Anregungsstrahlung unter 800 nm erzeugt wird und dass vermittels des Filters (10) im Kubus (3) ein bereinigte Raman- Signal über 800 nm erzeugt wird.

## Claims

1. Device for generating and detecting Red Flag radiation and a Raman spectrum comprising a laser light source (12) for generating Red Flag and Raman radiation in a biological tissue sample, a detector (18) for detecting a Red Flag overview image, a detection and control unit (16) for evaluating the Red Flag overview image and for controlling the Raman radiation, and a measuring probe (14) containing a bundle of light guides with at least one fibre (1) for placing on the surface of the biological tissue sample, a measuring probe (14) containing a bundle of optical fibres with at least one fibre (1) for placing on the surface of the biological tissue sample, wherein the device is arranged in such a way that the laser light can be coupled into one or more fibres (1) of the bundle of light guides and can be guided via a focusing element onto the surface of the biological tissue sample and light emitted from the tissue sample can be guided via the focusing element onto the detector (18) for Red Flag signal radiation and a Raman spectrometer (17) for Raman signal radiation, wherein the focusing element is arranged at the head of the measuring probe (14) in the form of a first transparent optical lens (2) and a second transparent optical lens (8) arranged directly adjacent thereto, by means of which the laser light of the fibre (1) can be coupled in collimated as Raman excitation radiation and the Raman signal radiation can be coupled out into the external fibre (9), wherein a transparent optical cube (3) with an adjoining transparent third optical lens (6) is arranged downstream of the first lens (2), so that the cube (3) is positioned directly between the first lens (2) and the third lens (6), wherein a filter layer (4), an absorber (5), a mirror (7) and a filter (10) are inserted in the cube (3) in the manner, that through the filter layer (4) the background generated in the fibre (1) can be directed onto the absorber (5) and excitation radiation can be guided through the third lens (6) onto the surface of the biological tissue sample, Raman signal radiation generated by the tissue sample can be collected and collimated through the third lens (6), the Raman excitation radiation and the Raman signal radiation can be separated by the filter (10) in the cube (3), so that the Raman signal radiation separated by the filter (10) can be deflected in the cube (3) by the mirror (7) and coupled via the second lens (8) into the external fibre (9), opening into the Raman spectrometer (17), wherein the first lens (2), the third lens (6) and the second lens (8) are graded index lenses, and the Red Flag radiation is an autofluorescence radiation, a two- or three-photon fluorescence radiation, a coherent anti-Stokes Raman radiation or a stimulated Raman scattering radiation.

2. Device according to claim 1, **characterized in that** the bundle of optical fibres with the fibre (1), the external fibre (9) and the measuring probe (14) have a substantially hollow cylindrical envelope, the end face of the third lens (6) not being enveloped and the inner diameter of the hollow cylinder substantially coinciding with the outer diameter of the bundle of optical fibres plus the fibre (9) and with the outer diameter of the measuring probe (14).

3. Device according to claim 2, **characterised in that** the enclosure is a channel of an endoscope and the bundle of optical fibres with the at least one fibre (1) as well as the external fibre (9) are combined into a fibre combination bundle (13).

4. Device according to claim 1, **characterized in that** the laser light source is a Nd-YAG laser, a He-Ne laser or a semiconductor laser.

5. Device according to claim 1, **characterised in that** the detector (18) is a photo-optical detector by means of which the red-flag overview image is detectable.

6. Device according to one or more of the preceding claims 1 to 5, **characterized in that** the detection and control unit (16) comprises a computer containing software for evaluating the red-flag overview image generated by the detector (18), the Raman excitation radiation being selectively addressable by the detection and control unit (16), individually into at least one of the fibres (1) of the bundle of optical fibres (13) by the radiation of the laser light source (12).

7. Device according to claim 1, **characterized in that** the diameter of the external fibre (9) is equal to the diameter of the fibre (1), in which the external fibre (9) is formed by a cross-section transducer in which 19 MM fibres are distributed as a linear or circular array individually and substantially uniformly around the outer periphery of the fibre (1) and supported by the latter, the sum of the individual diameters of the 19 MM fibres being equal to the diameter of the fibre (1).

8. Method for generating and detecting Red Flag radiation and a Raman spectrum using a device according to one or more of the preceding claims 1 to 7 in which
- by means of the laser light source (12) a Red-Flag excitation radiation is guided via the bundle of light guides with at least one fibre (1) onto the surface of a biological tissue sample,
- red-flag signal radiation emitted by the sample is guided onto the detector (18),
- the signals detected by the detector (18) as a red-flag overview image are evaluated with computer support in the detection and control unit (16), the Raman excitation radiation from the laser light source (12) being introduced individually into a fibre (1) of the bundle of light guides (13) on the basis of the evaluated Raman excitation radiation which can be addressed in a targeted manner by means of the detection and control unit (16),
- this Raman excitation radiation is guided via the fibre (1), through the first lens (2), the filter layer (4) of the cube (3) and the third lens (6) onto the surface of the biological tissue sample,
- Raman signal radiation emitted by the sample is guided from the third lens (6) via the filter layer (4) of the cube (3), through the filter (10) via the mirror (7) through the second lens (8) into the external fibre (9),
- the Raman signal radiation is guided from the fibre (9) into the Raman spectrometer (17) and - structural parameters of the tissue are determined from the Raman spectrum of the specifically selected section of the surface of the biological tissue sample detected in the Raman spectrometer (17), which provide conclusions about the properties of the tissue section to be characterised.

9. A method according to claim 8, **characterized in that** a cleaned Raman excitation radiation below 800 nm is generated by means of the filter layer (4) and the adsorber (5) in the cube (3) and that a cleaned Raman signal above 800 nm is generated by means of the filter (10) in the cube (3).

## Revendications

1. Dispositif pour la génération et la détection d'un rayonnement Red-Flag et d'un spectre Raman comprenant une source de lumière laser (12) pour la génération d'un rayonnement Red-Flag et d'un rayonnement Raman dans un échantillon de tissu biologique, un détecteur (18) pour la détection d'une image d'ensemble Red-Flag, une unité de détection et de commande (16) pour l'évaluation de l'image d'ensemble Red Flag et pour la commande du rayonnement d'excitation Raman, ainsi qu'une sonde de mesure (14) contenant un faisceau de guides de lumière avec au moins une fibre (1), destinée à être placée sur la surface de l'échantillon de tissu biologique, le dispositif étant agencé de telle sorte, que la lumière laser peut être couplée dans une ou plusieurs fibres (1) du faisceau de fibres optiques et peut être guidée par l'intermédiaire d'un élément de focalisation sur la surface de l'échantillon de tissu biologique et que la lumière émise par l'échantillon de tissu peut être guidée par l'intermédiaire de l'élément de focalisation sur le détecteur (18) pour le rayonnement de signal de drapeau rouge et un spectromètre Raman (17) pour le rayonnement de signal Raman, l'élément de focalisation étant disposé à la tête de la sonde de mesure (14) sous la forme d'une première lentille optique transparente (2) et d'une deuxième lentille optique transparente (8) disposée directement à côté, au moyen desquelles la lumière laser de la fibre (1) peut être injectée sous forme de rayonnement d'excitation Raman collimaté et le rayonnement de signal Raman peut être découplé dans 1a fibre externe (9), un cube optique transparent (3) suivi d'une troisième lentille optique transparente (6) étant disposé en aval de 1a première lentille (2), de sorte que le cube (3) est positionné directement entre la première lentille (2) et la troisième lentille (6), une couche filtrante (4), un absorbeur (5), un miroir (7) et un filtre (10) étant introduits dans le cube (3) de telle sorte, la couche filtrante (4) permet de diriger le fond formé dans la fibre (1) sur l'absorbeur (5) et le rayonnement d'excitation peut être guidé à travers la troisième lentille (6) sur la surface de l'échantillon de tissu biologique, le rayonnement de signal Raman généré par l'échantillon de tissu peut être collecté et collimaté par la troisième lentille (6), le rayonnement d'excitation Raman et le rayonnement de signal Raman peuvent être séparés par le filtre (10) dans le cube (3), de sorte que le rayonnement de signal Raman séparé par le filtre (10) peut être dévié dans le cube (3) par le miroir (7) et peut être couplé par la deuxième lentille (8) dans la fibre externe (9) qui débouche dans le spectromètre Raman (17), la première lentille (2), la troisième lentille (6) et la deuxième lentille (8) étant réalisées sous forme de lentilles à gradient d'indice, et le rayonnement du drapeau rouge étant un rayonnement d'autofluorescence, un rayonnement de fluorescence à deux ou trois photons, un rayonnement cohérent anti-Stokes-Raman ou un rayonnement de diffusion Raman stimulé.

2. Dispositif selon la revendication 1, **caractérisé en ce que** le faisceau de fibres optiques comprenant la fibre (1), la fibre externe (9) et la sonde de mesure (14) présentent une enveloppe sensiblement cylindrique creuse, la face frontale de la troisième lentille (6) n'étant pas enveloppée et le diamètre intérieur du cylindre creux coïncidant sensiblement avec le diamètre extérieur du faisceau de fibres optiques plus la fibre (9) ainsi qu'avec le diamètre extérieur de la sonde de mesure (14).

3. Dispositif selon la revendication 2, **caractérisé en ce que** l'enveloppe est un canal d'un endoscope et le faisceau de fibres optiques comprenant la au moins une fibre (1) ainsi que la fibre externe (9) sont réunis en un faisceau de fibres combiné (13).

4. Dispositif selon la revendication 1, **caractérisé en ce que** la source de lumière laser est un laser Nd-YAG, un laser He-Ne ou un laser à semi-conducteur.

5. Dispositif selon la revendication 1, **caractérisé en ce que** le détecteur (18) est un détecteur photo-optique au moyen duquel l'image d'ensemble du drapeau rouge peut être détectée.

6. Dispositif selon l'une ou plusieurs des revendications 1 à 5 ci-dessus, **caractérisé en ce que** l'unité de détection et de commande (16) comprend un ordinateur contenant un logiciel pour l'évaluation de l'image d'ensemble du drapeau rouge générée par le détecteur (18), le rayonnement d'excitation Raman pouvant être adressé de manière ciblée par l'unité de détection et de commande (16), généré individuellement dans au moins une des fibres (1) du faisceau de fibres optiques (13) par le rayonnement de la source de lumière laser (12).

7. Dispositif selon la revendication 1, **caractérisé en ce que** le diamètre de la fibre externe (9) est égal au diamètre de la fibre (1), dans lequel la fibre externe (9) est formée par un convertisseur de section transversale dans lequel 19 fibres MM sont disposées de manière linéaire ou circulaire, individuellement et de manière sensiblement uniforme autour de la circonférence externe de la fibre (1).

8. Procédé de génération et de détection d'un rayonnement Red-Flag et d'un spectre Raman en utilisant un dispositif selon une ou plusieurs des revendications 1 à 7 ci-dessus, dans lequel
- au moyen de la source de lumière laser (12), un rayonnement d'excitation Red Flag est guidé sur la surface d'un échantillon de tissu biologique par le biais du faisceau de fibres optiques avec au moins une fibre (1),
- le rayonnement de signal Red-Flag émis par l'échantillon est dirigé vers le détecteur (18),
- les signaux saisis par le détecteur (18) sous la forme d'une image d'ensemble du drapeau rouge sont évalués par ordinateur dans l'unité de détection et de commande (16), le rayonnement d'excitation Raman de la source de lumière laser (12) pouvant être adressé de manière ciblée, sur la base des signaux évalués, dans une fibre (1) du faisceau de fibres optiques (13),
- ce rayonnement d'excitation Raman est guidé sur la surface de l'échantillon de tissu biologique par la fibre (1), à travers la première lentille (2), la couche filtrante (4) du cube (3) et la troisième lentille (6),
- le rayonnement de signal Raman émis par l'échantillon est guidé par la troisième lentille (6), à travers la couche filtrante (4) du cube (3), à travers le filtre (10), à travers le miroir (7), à travers la deuxième lentille (8), dans la fibre externe (9),
- le rayonnement de signal Raman est conduit de la fibre (9) dans le spectromètre Raman (17) et
- à partir du spectre Raman détecté dans le spectromètre Raman (17) de la section examinée sélectionnée de manière ciblée à partir de la surface de l'échantillon de tissu biologique, on détermine des paramètres structurels du tissu qui fournissent des conclusions sur les propriétés de la section de tissu à caractériser.

9. Procédé selon la revendication 8, **caractérisé en ce que**, au moyen de la couche filtrante (4) et de l'adsorbeur (5) dans le cube (3), on produit un rayonnement d'excitation Raman épuré inférieur à 800 nm et **en ce que**, au moyen du filtre (10) dans le cube (3), on produit un signal Raman épuré supérieur à 800 nm.
